Europäisches Patentamt

European Patent Office

Office européen des brevets

(19)

(11) EP 0 564 369 B1

(12) FASCICULE DE BREVET EUROPEEN

(45) Date de publication et mention
de la délivrance du brevet:
**02.10.2002 Bulletin 2002/40**

(51) Int Cl.⁷: $A61L\ 27/00$

(21) Numéro de dépôt: **93400862.4**

(22) Date de dépôt: **02.04.1993**

(54) **Matériau pour prothèse osseuse contenant des particules de carbonate de calcium dispersées dans une matrice polymère biorésorbable**

Material für Knochenprothese enthaltend in einer bioresorbierbaren Polymermatrix dispergierte Calciumcarbonatteilchen

Material for bone prosthesis containing calcium carbonate particles dispersed in a bioresorbable polymeric matrix

(84) Etats contractants désignés:
**AT BE CH DE DK ES GB GR IE IT LI LU NL SE**

(30) Priorité: **03.04.1992 FR 9204096**

(43) Date de publication de la demande:
**06.10.1993 Bulletin 1993/40**

(73) Titulaire: **BIO HOLDINGS INTERNATIONAL LIMITED**
**Tortola (VG)**

(72) Inventeurs:
• **Christel, Pascal**
  **F-75018 Paris (FR)**
• **Li, Su Ming**
  **Les Cévennes, F-34080 Montpellier Cedex (FR)**
• **Vert, Michel**
  **F-34170 Castelnau-Le-Lez (FR)**
• **Patat, Jean-Louis**
  **F-75017 Paris (FR)**

(74) Mandataire: **Hubert, Philippe**
**Cabinet Beau de Loménie**
**158, rue de l'Université**
**75340 Paris Cédex 07 (FR)**

(56) Documents cités:
  FR-A- 2 364 644          FR-A- 2 460 657
  US-A- 4 722 948

**Description**

La présente invention a pour objet un matériau pour prothèse osseuse biorésorbable contenant des particules de carbonate de calcium dispersées au sein d'une matrice polymère d'acide lactique.

On sait que certains polymères tels que les polyesters aliphatiques, et en particulier ceux qui sont dérivés des acides lactique et glycolique, ont été préconisés comme matériaux biorésorbables, par exemple en chirurgie, pour la réalisation de fils de suture ou de pièces d'ostéosynthèse (voir par exemple les brevets US 3.739.773 et 3.867.190, et les brevets FR 2.364.644 et 2.439.003), ou encore en pharmacologie pour la réalisation de système de libération contrôlée de principes actifs (voir par exemple le brevet FR-2.070.153). Ce sont des polymères synthétiques biorésorbables, c'est-à-dire qu'ils sont progressivement dégradés dans l'organisme et éliminés par les voies naturelles. Leurs produits de dégradation, par exemple les acides lactique et glycolique, sont des métabolites normaux et sont donc parfaitement tolérés par le milieu vivant.

Dans le brevet FR 2.460.657, on a décrit l'utilisation de carbonate de calcium, notamment sous la forme de pièces massives, dans la réalisation d'implants biodégradables utilisables comme pièces de prothèse osseuse. De tels implants, réalisés par exemple à partir de squelettes de corail naturel, sont bien tolérés et leur dégradation progressive s'effectue au profit d'une repousse du tissu osseux.

L'un des inconvénients des pièces de prothèse à base de carbonate de calcium est leur difficulté de mise en forme qui peut nécessiter des usinages délicats dans le cas de formes complexes.

Par ailleurs, un inconvénient des polyesters d'hydroxyacides mentionnés ci-dessus est que leur vitesse de dégradation est difficile à contrôler. Cette vitesse de dégradation est généralement trop lente dans le cas où les polymères sont à l'état cristallin, et est souvent trop rapide lorsque les polymères sont à l'état amorphe.

En outre, on a découvert que la dégradation est plus rapide à l'intérieur du matériau polymère qu'en surface, ce qui peut fragiliser dangereusement la pièce de prothèse implantée.

Dans le brevet FR 2.364.644, on avait décrit plus particulièrement l'utilisation de polymères cristallins avec introduction, dans le matériau polymère, d'une charge (phosphate de calcium) présente à raison de 0,5 à 30 % en poids, et de préférence de 0,5 à 5 % en poids, de façon à faciliter la résorption du polymère. Le mécanisme suggéré était que la présence de la charge crée dans la masse polymère des microhétérogénéités facilitant l'attaque en certains points du matériau polymère et modifiant donc sa capacité de résorption. Autrement dit, on considérait que les particules de la charge constituaient des points d'attaque préférentiels de la surface du polymère, avec constitution de microcavités favorables au développement de tissu osseux néoformé.

On a maintenant découvert que, de façon surprenante, l'association de particules de carbonate de calcium à un polymère d'acide lactique permet de diminuer la vitesse de résorption du polymère, contrairement à ce qui était observé, dans le cas d'une charge à base de phosphate de calcium, dans le brevet FR 2.364.644.

En outre, les propriétés de l'implant polymère, grâce à l'incorporation de particules de carbonate de calcium, sont notablement améliorées, comme cela est précisé dans la partie expérimentale ci-après.

On a également découvert qu'il est généralement préférable d'utiliser, dans la réalisation de pièces de prothèse osseuse, des polymères amorphes.

Le carbonate de calcium améliore les propriétés de l'implant, même lorsque le matériau polymère est un matériau cristallin.

Grâce à l'association de quantités importantes de carbonate de calcium particulaire avec une matrice polymère, il est possible d'obtenir un matériau composite qui présente les mêmes avantages que les matériaux à base de carbonate de calcium, sans en présenter les inconvénients, c'est-à-dire la difficulté de réaliser des pièces de forme quelconque.

La présente invention a donc pour objet un matériau pour prothèse osseuse biorésorbable, contenant des particules de carbonate de calcium dispersées au sein d'une matrice polymère, lesdites particules ayant des dimensions inférieures à 1 mm et représentant de 40 à 70 % du poids total, et ledit polymère étant un polymère d'acide lactique.

Les polymères utilisables selon l'invention sont bien entendu des polymères biocompatibles, c'est-à-dire implantables chez l'homme ou chez les animaux vertébrés sans provoquer sur l'organisme des effets secondaires inacceptables. De tels polymères sont bien connus, de nombreux représentants ayant été décrits dans la littérature.

Ces polymères peuvent être des homopolymères ou des copolymères.

De préférence, on utilise des polymères amorphes.

Parmi les polymères utilisables dans la réalisation du matériau selon l'invention, on citera plus particulièrement les poly(acides lactiques) provenant de la polymérisation de mélanges de L- et de D-lactides en proportions telles que lesdits poly(acides lactiques) soient amorphes. Ces polymères sont constitués d'un mélange de motifs dérivés des acides D- et L-lactiques. On peut utiliser notamment un poly(acide lactique) contenant de 20 à 80 %, et en particulier de 30 à 70 %, en motifs, de motifs D-lactiques. On peut utiliser à particulier un poly(acide lactique) contenant en proportions égales des motifs dérivés des acides D- et L-lactiques.

Parmi les copolymères utilisables, on peut citer en particulier les copolymères constitués de motifs dérivés d'acides lactique et glycolique, et par exemple un copolymère contenant jusqu'à 50 %, en motifs, de motifs dérivés d'acide glycolique. Avec plus de 50 % de motifs glycoliques, les copolymères sont difficiles à purifier car ils deviennent insolubles dans les solvants usuels.

Lorsque la pièce de prothèse est destinée à remplacer des parties d'os subissant des contraintes relativement importantes, elle doit avoir des propriétés mécaniques suffisantes. Il convient en général que le polymère ait une masse moléculaire moyenne suffisamment élevée, par exemple une masse moléculaire en poids au moins égale à 40000 environ, pour que le matériau de l'invention soit capable de supporter les contraintes mécaniques auxquelles il doit être soumis après implantation. Le choix de la masse moléculaire peut donc être déterminé dans chaque cas par des expériences de routine.

Lorsque la pièce de prothèse est une pièce de comblement ne subissant pas de contraintes mécaniques importantes, il est possible d'utiliser le matériau polymère sous la forme d'un produit pâteux qui peut être facilement mis en place en raison de son caractère modelable. Le matériau pâteux peut être obtenu en utilisant des polymères amorphes de faible masse moléculaire moyenne, ou en utilisant un mélange de polymères amorphes de haute et faible masses moléculaires moyennes. Par exemple, il est possible d'obtenir des polymères ou copolymères pâteux, notamment en mélangeant un poly(acide lactique) amorphe, de masse moléculaire moyenne élevée, par exemple supérieure à 20000, avec un poly(acide lactique) amorphe de masse moléculaire moyenne faible (en particulier inférieure à 10000, ou inférieure à 5000), ce dernier jouant le rôle de plastifiant. La proportion de poly(acide lactique) de faible masse moléculaire pourra être en particulier une proportion suffisante pour que la température de transition vitreuse du mélange de polymères soit inférieure à une température prédéterminée, en particulier inférieure à 50°C, par exemple à 37°C. En effet, la température de transition vitreuse diminue lorsque la proportion de polymère de faible masse moléculaire augmente.

On peut régler la souplesse de la pâte, qui augmente avec la teneur en poly(acide lactique) de faible masse moléculaire. Ici encore, la composition du mélange polymère peut donc être déterminée par de simples expériences de routine.

Les polymères mentionnés ci-dessus sont connus ou peuvent être préparés selon les méthodes connues. Certains d'entre-eux sont d'ailleurs des produits commerciaux. Par exemple, pour préparer un poly(acide lactique) de masse moléculaire moyenne élevée, ou un copolymère lactique-glycolique, on peut opérer par ouverture de cycle du diester cyclique (lactide, glycolide) selon les méthodes usuelles. Pour préparer un poly(acide lactique) de faible masse moléculaire, on peut opérer notamment par polycondensation de mélanges d'acides L et D-lactiques, par exemple d'acide D,L-lactique. Des copolymères correspondants sont préparés de façon analogue.

Les masses moléculaires des polymères utilisés selon l'invention peuvent être déterminées par exemple en solution, par chromatographie par perméation de gel, par comparaison avec des polymères étalons (par exemple polystyrène).

Le matériau de l'invention peut contenir le carbonate de calcium sous forme cristalline (aragonite et/ou calcite). Le carbonate de calcium peut être obtenu à partir de tout matériau, naturel ou synthétique, poreux ou non poreux, contenant de la calcite ou de l'aragonite.

Parmi les matériaux à base d'aragonite, on citera notamment ceux constitués par du squelette de corail. On peut utiliser par exemple du squelette de corail madréporaire tel que le Porites, le Pocillopora ou le Favites.

On peut également utiliser les coquilles de certains mollusques ou bivalves, par exemple Pinctada margaritifera.

Les matériaux à base de calcite peuvent être constitués notamment par des squelettes d'échinodermes, en particulier des squelettes d'oursins ou encore des épines d'oursins.

Les squelettes de corail ou d'échinodermes peuvent être débités en tronçons puis soumis à un broyage et éventuellement à un tamisage pour obtenir des particules ayant les dimensions souhaitées. Les particules peuvent ensuite être soumises à un traitement permettant d'éliminer les résidus organiques, par exemple par immersion dans une solution d'hypochlorite de sodium pendant 48h et ensuite rincés à l'eau courante puis stérilisés, par exemple à la chaleur humide (120°C, 30 min).

Pour préparer le matériau de l'invention on peut opérer par mélange du polymère constituant la matrice, sous forme de poudre ou de pâte, avec la poudre de carbonate de calcium.

On peut également opérer en ajoutant la poudre de carbonate de calcium dans une solution du polymère dans un solvant organique, après quoi on évapore le solvant (par exemple l'acétone) sous agitation. On peut ensuite éliminer le solvant résiduel dans une étuve à vide. Le mélange solide obtenu peut être ensuite moulé par compression à une température au moins égale à la température de ramollissement du polymère.

On peut également soumettre le mélange solide obtenu à un broyage, de façon à obtenir le matériau de l'invention sous forme de poudre. La poudre obtenue, qui a par exemple des dimensions de particules de 50-500 μm, peut ensuite être mise sous la forme désirée par moulage.

Les pièces massives moulées peuvent en outre être usinées pour leur donner sensiblement la forme du fragment osseux qu'elles sont destinées à remplacer.

Le matériau de l'invention peut être utilisé en chirurgie osseuse, y compris en chirurgie dentaire (par exemple pour la réalisation de racines dentaires artificielles).

Le matériau de l'invention sous forme de poudre peut également être utilisé pour revêtir, par moulage, des pièces massives en matériau inerte, telles que la queue fémorale d'une prothèse de hanche, ce qui permet la fixation temporaire, par emmanchement à force, dans la cavité médullaire, puis favorise la fixation définitive par stimulation de la repousse osseuse au contact de l'implant. Ce revêtement de prothèse permet de remplacer l'interface de ciment acrylique généralement utilisé, qui constitue la source majeure des descellements observés dans ce genre d'opération.

L'invention a également pour objet les pièces de prothèse osseuse constituées, totalement ou partiellement, à base du matériau de l'invention.

L'invention a également pour objet l'utilisation de particules de carbonate de calcium dispersées au sein d'une matrice polymère, dans la préparation d'une pièce de prothèse osseuse, lesdites particules ayant des dimensions inférieures à 1 mm et ledit polymère étant un d'acide lactique, tel que défini ci-dessus.

L'invention a en outre pour objet un procédé pour améliorer les propriétés des prothèses osseuses contenant un polymère biodégradable, par incorporation de carbonate de calcium dans une matrice dudit polymère, comme décrit ci-dessus.

On peut bien entendu incorporer dans la matrice polymère d'autres ingrédients actifs usuels sous forme particulaire, par exempte des antibiotiques.

Les exemples suivants illustrent l'invention sans toutefois la limiter.

**EXEMPLE 1:** *Synthèse, purification et caractérisation de polymère*

Les poly(acides lactiques) sont désignés par la dénomination PLA X (X représentant le pourcentage des motifs dérivés de l'acide L-lactique).

Les copolymères acide lactique/acide glycolique sont désignés par la dénomination PLA X GA Y, X représentant comme précédemment le pourcentage de motifs dérivés d'acide L-lactique et Y représentant le pourcentage des motifs dérivés d'acide glycolique, dans le mélange de monomères de départ. La teneur de ces polymères en motifs dérivés d'acide D-lactique est donc (100-X-Y). Les pourcentages sont ici des pourcentages en motifs.

Les polymères n° 1 et 2 sont commercialisés par la Société Phusis.

On a préparé le polymère n°3 par ouverture de cycle du D,L-lactide (100 g) recristallisé dans l'acétone puis séché sous vide (45°C; 3 jours). La polymérisation est effectuée à 140°C pendant 8 jours, en présence de 0,05 % en poids de poudre de zinc. Le polymère brut obtenu est purifié par dissolution dans 500 ml d'acétone, puis précipitation par addition progressive d'éthanol. Le polymère précipité est séché sous vide (40°C ; 8 jours).

Les polymères commerciaux ont été soumis à une purification analogue.

La caractérisation des polymères a été effectuée par chromatographie par perméation de gel (GPC) par comparaison avec des étalons de polystyrène, la phase mobile étant le dioxanne.

On donne dans le tableau (I) les masses moléculaires des polymères préparés.

TABLEAU I

| Polymère n° | 1 | 2 | 3 |
|---|---|---|---|
| Composition | PLA37,5 GA25 | PLA50 | PLA50 |
| $\bar{M}p(x10^{-3})$ | 63 | 136 | 228 |

**EXEMPLE 2:** *Elaboration de mélanges corail/polymère*

Le corail, sous forme de poudre, est celui commercialisé par la Société Inoteb sous la dénomination Biocoral (granulométrie : 300-450 µm).

a) *Mélange à l'air*

Dans 18 ml d'acétone, on ajoute 4 g du polymère n°1 et on agite jusqu'à dissolution. On ajoute ensuite 6 g de poudre de corail. En maintenant l'agitation, on évapore progressivement le solvant Le mélange est ensuite séché dans une étuve à vide à 40°C pour éliminer le solvant résiduel.

Le mélange a été moulé par compression de la manière suivante : le moule est posé sur le plateau chauffant (150°C) d'une presse. Après 30 minutes, le moule, ayant atteint une température d'équilibre de 132°C environ, on introduit le mélange dans le moule et chauffe pendant 10 minutes. On applique ensuite une pression de 100 bars

($10^7$Pa) puis on refroidit rapidement le moule par circulation d'eau froide pendant 10 minutes. Après ouverture du moule froid, on obtient une plaque de 75 mm de diamètre et 1,2 mm d'épaisseur.

De la même façon, on a préparé un cylindre ayant un diamètre de 14 mm et une hauteur de 9 mm.

De façon analogue, on a préparé des plaques et cylindres avec les polymères n°2 et n°3 et une proportion de corail égale, comme précédemment, à 60 %.

b) *Mélange sous vide*

On mélange 2 g du polymère n°2 et 10 ml d'acétone dans un ballon relié à un évaporateur rotatif. Lorsque le vide est établi, on verse 3 g de corail dans la solution. Le ballon est laissé en rotation lente pour obtenir un mélange homogène tandis que le solvant est évaporé progressivement. Le mélange est ensuite séché dans une étuve à vide à 40°C pendant 24 h. Avec ce mélange, on a préparé par moulage une plaque et un cylindre, comme précédemment.

On constate que pour une même masse de polymère et de corail, le mélange préparé sous vide a un volume moindre que le mélange préparé à l'air. L'explication de ce phénomère est que le corail utilisé ici comme source de carbonate de calcium est poreux.

Dégradation *in vitro*

Les plaques obtenues précédemment ont été découpées en petites éprouvettes de 12 mm x 12 mm environ. Chaque éprouvette a été introduite dans un flacon rempli de 30 ml de tampon phosphate (pH = 7,4). Les flacons sont placés dans une étuve à 37°C. A chaque intervalle de temps, des éprouvettes sont prélevées, rincées, séchées et analysées.

On a procédé notamment à la comparaison d'éprouvettes provenant d'une plaque réalisée comme précédemment avec le polymère n°3 seul (sans corail), et avec le polymère n°3 et le corail (mélange à 60 % en poids de corail). On a comparé des éprouvettes de même poids.

Tout d'abord, il a été constaté par l'examen visuel que les éprouvettes de PLA50 ont beaucoup gonflé et se sont déformées en cours de dégradation, alors que celles du mélange corail/PLA 50 ont conservé la même forme et les mêmes dimensions.

Pour le PLA50, le taux d'eau absorbée a augmenté très rapidement et a atteint une valeur de 176 % au bout de 10 semaines (gonflement des éprouvettes). Dans le cas du mélange corail/PLA50, le taux de l'eau absorbée était plus important (environ 40 %) que pour le PLA50 après une semaine de dégradation, mais ensuite le taux de l'eau absorbée n'a plus augmenté : il avait même tendance à décroître, pour atteindre moins de 30 % après 25 semaines.

Pour le PLA50, il n'y a pas eu de perte de masse détectable avant 10 semaines. Entre 10 et 17 semaines, une forte perte (73 %) a été observée. Au-delà, les éprouvettes ont continué à perdre de la masse mais plus lentement. Par contre, le cas du mélange corail/PLA50 est très différent. Après une semaine, une faible perte de 2 % est détectée. Par la suite, la perte de masse n'a augmenté que très légèrement pour atteindre 3 ou 4 % au bout de 25 semaines.

On a également étudié l'évolution de la concentration en acide L-lactique détecté dans le milieu de dégradation. Cette évolution coïncide parfaitement avec celle de la perte de masse. Avec le PLA50, il n'y a pas d'acide L-lactique détecté jusqu'à 10 semaines. Entre 10 et 17 semaines, une forte augmentation de la concentration en acide L-lactique a été observée. Au-delà cette augmentation s'est ralentie. Pour le mélange PLA50/corail, la concentration en acide lactique est environ 5 fois plus faible (17 semaines) et 10 fois plus faible (25 semaines) que dans le cas du PLA50 seul.

En conclusion, l'incorporation de corail ralentit la dégradation du polymère, et modifie les caractéristiques de cette dégradation.

**Revendications**

1. Matériau pour prothèse osseuse biorésorbable, contenant des particules de carbonate de calcium dispersées au sein d'une matrice polymère, lesdites particules ayant des dimensions inférieures à 1 mm et représentant de 40 à 70 % du poids total, et ledit polymère étant un polymère d'acide lactique.

2. Matériau selon la revendication 1, **caractérisé par le fait que** ladite matrice est en polymère amorphe.

3. Matériau selon la revendication 2, **caractérisé par le fait que** ledit polymère est un poly(acide lactique) constitué d'un mélange de motifs dérivés des acides D- et L-lactiques, les proportions de chacun des motifs D- et L- étant suffisantes pour que ledit poly(acide lactique) soit amorphe.

**4.** Matériau selon la revendication 3, **caractérisé par le fait que** ledit poly(acide lactique) contient de 30 à 70 % de motifs D-lactiques.

**5.** Matériau selon la revendication 4, **caractérisé par le fait que** ledit poly(acide lactique) contient en proportions égales des motifs dérivés des acides D- et L-lactiques.

**6.** Matériau selon la revendication 1 ou 2, **caractérisé par le fait que** ledit polymère est un copolymère.

**7.** Matériau selon la revendication 6, **caractérisé par le fait que** ledit copolymère est un copolymère constitué de motifs dérivés d'acides lactiques et d'acide glycolique.

**8.** Matériau selon la revendication 7, **caractérisé par le fait que** ledit copolymère contient jusqu'à 50 %, en motifs, de motifs dérivés d'acide glycolique.

**9.** Matériau selon l'une quelconque des revendications 1 à 8, **caractérisé par le fait que** ledit polymère a une masse moléculaire moyenne suffisamment élevée pour que le matériau soit capable de supporter les contraintes mécaniques auxquelles il doit être soumis après implantation.

**10.** Matériau selon la revendication 9, **caractérisé par le fait que** ledit polymère a une masse moléculaire moyenne en poids au moins égale à 40000 environ.

**11.** Matériau selon l'une quelconque des revendications 1 à 8, **caractérisé par le fait que** ledit polymère a une masse moléculaire moyenne suffisamment faible pour **que** le matériau se présente sous la forme d'un solide pâteux à température inférieure à 50°C, et en particulier à 37°C.

**12.** Matériau selon la revendication 11, **caractérisé par le fait qu'**il est constitué au moins en partie d'un polymère ayant une masse moléculaire moyenne en poids inférieure à 10000, et en particulier inférieure à 5000.

**13.** Matériau selon l'une quelconque des revendications 1 à 12, **caractérisé par le fait qu'**il contient de 50 à 70 % en poids de carbonate de calcium.

**14.** Matériau selon l'une quelconque des revendications 1 à 13, **caractérisé par le fait que** ledit carbonate de calcium est sous forme de calcite ou d'aragonite.

**15.** Matériau selon l'une quelconque des revendications 1 à 14, **caractérisé par le fait que** ledit carbonate de calcium est obtenu à partir de squelettes d'organisme marin à base de carbonate de calcium, ou de coquilles de mollusques ou de bivalves.

**16.** Matériau selon la revendication 15, **caractérisé par le fait que** ledit carbonate de calcium est obtenu à partir de squelettes de corail, de squelettes ou épines d'oursins, ou de coquilles de Pinctada margaritifera.

**17.** Matériau selon l'une quelconque des revendications 1 à 16, **caractérisé par le fait qu'**il se présente sous la forme d'une poudre moulable ou sous la forme d'une pièce massive moulée.

**18.** Utilisation de particules de carbonate de calcium dispersées au sein d'une matrice polymère dans des proportions de 40 à 70% en poids du poids total, dans la préparation d'une pièce de prothèse osseuse, lesdites particules ayant des dimensions inférieures à 1 mm, et ledit polymère étant un polymère d'acide lactique.

**19.** Utilisation selon la revendication 18, **caractérisée par le fait que** ledit matériau est tel que défini dans l'une quelconque des revendications 2 à 17.

**20.** Procédé pour diminuer la vitesse de résorption d'un polymère d'acide lactique formant matrice d'un matériau pour prothèse osseuse, **caractérisé par le fait que** l'on disperse au sein de ladite matrice des particules de carbonate de calcium, lesdites particules ayant des dimensions inférieures à 1 mm et représentant de 40 à 70 % du poids total de la matrice.

**21.** Procédé selon la revendication 20, **caractérisé par le fait que** ladite matrice polymère est telle que définie dans l'une quelconque des revendications 2 à 12.

**Claims**

1. Bioresorbable bone prosthesis material containing calcium carbonate particles dispersed within a polymer matrix, said particles being smaller than 1 mm in size and representing from 40 to 70% of the total weight, and said polymer being a lactic acid polymer.

2. Material according to claim 1, **characterized in that** said matrix is made of an amorphous polymer.

3. Material acording to claim 2, **characterized in that** said polymer is a poly(lactic acid) consisting of a mixture of units derived from D- and L-lactic acids, the proportions of each of the D and L units being sufficient for the said poly(lactic acid) to be amorphous.

4. Material according to the claim 3, **characterized in that** said poly(lactic acid) contains from 30 to 70% of D-lactic units.

5. Material according to the claim 4, **characterized in that** said poly(lactic acid) contains equal proportions of the units derived from D- and L-lactic acids.

6. Material according to claim 1 or 2, **characterized in that** said polymer is a copolymer.

7. Material according to claim 6, **characterized in that** said copolymer is a copolymer containing units derived from lactic acids and from glycolic acid.

8. Material according to claim 7, **characterized in that** said copolymer contains up to 50%, expressed in terms of units, of units derived from glycolic acid.

9. Material according to any one of claims 1 to 8, **characterized in that** said polymer has a sufficiently high average molecular mass for the material to be capable of withstanding the mechanical stresses to which it has to be subjected after implantation.

10. Material according to claim 9, **characterized in that** said polymer has a weight average molecular mass equal to at least about 40,000.

11. Material according to any one of claims 1 to 8, **characterized in that** said polymer has a sufficiently low average molecular mass for the material to take the form of a pasty solid at a temperature below 50°C, in particular at 37°C.

12. Material according to claim 11, **characterized in that** said polymer consists at least partially of a polymer having a weight average molecular mass of less than 10,000, in particular less than 5,000.

13. Material according to any one of claims 1 to 12, **characterized in that** said material contains from 50 to 70% by weight of calcium carbonate.

14. Material according to any one of claims 1 to 13, **characterized in that** said calcium carbonate is in the form of calcite or aragonite.

15. Material according to any one of claims 1 to 14, **characterized in that** said calcium carbonate is obtained from calcium carbonate-based marine organism skeletons, from mollusk shells or from bivalve shells.

16. Material according to claim 15, **characterized in that** said calcium carbonate is obtained from coral skeletons, from sea urchin skeletons or spines or from Pinctada margaritifera shells.

17. Material according to any one of claims 1 to 16, **characterized in that** said material is in the form of a moldable powder or in the form of a molded solid item.

18. Use of calcium carbonate particles dispersed within a polymer matrix in propertions of 40 to 70 % by weight of the total weight, in the preparation of a bone prosthesis item, said particles being smaller than 1 mm in size, and said polymer being a polymer of lactic acid.

**19.** Use according to claim 18, **characterized in that** said material is as defined in any one of claims 2 to 17.

**20.** A method of decreasing the rate of resorption of a polymer of lactic acid forming a matrix of a bone prosthesis material, **characterized in that** calcium carbonate particles are dispersed within said matrix, said particles having a size of less than 1 mm and representing 40 to 70% of the total weight of the matrix.

**21.** A method according to claim 20, **characterized in that** said polymer matrix is as defined in any one of claims 2 to 12.

**Patentansprüche**

**1.** Bioresorbierbares Material für eine Knochenprothese, die Calciumcarbonatpartikel enthält, innerhalb einer Polymermatrix verteilt sind, wobei die Partikel Größen unter 1 mm haben und 40 bis 70% des Gesamtgewichts darstellen, und das Polymer ein Milchsäurepolymer ist.

**2.** Material gemäß Anspruch 1, **dadurch gekennzeichnet, dass** die Matrix aus einem amorphen Polymer besteht.

**3.** Material gemäß Anspruch 2, **dadurch gekennzeichnet, dass** das Polymer eine Poly(Milchsäure) ist, das aus einer Mischung von Motiven besteht, die von D- und L-Milchsäure abstammen, wobei die Anteile jedes der Motive D- und L-ausreichend sind, damit die Poly(Milchsäure) amorph ist.

**4.** Material gemäß Anspruch 3, **dadurch gekennzeichnet, dass** die Poly(Milchsäure) 30 bis 70% an D-Milchsäuremotiven enthält.

**5.** Material gemäß Anspruch 4, **dadurch gekennzeichnet, dass** die Poly(Milchsäure) gleiche Anteile der Motive enthält, die von den D- und L-Milchsäuren abstammen.

**6.** Material gemäß Anspruch 1 oder 2, **dadurch gekennzeichnet, dass** das Polymer ein Copolymer ist.

**7.** Material gemäß Anspruch 6, **dadurch gekennzeichnet, dass** das Copolymer ein Copolymer ist, das aus Motiven besteht, die von den Milchsäuren und Glykolsäure abstammen.

**8.** Material gemäß Anspruch 7, **dadurch gekennzeichnet, dass** das Copolymer bis zu 50% der Motive an Motiven enthält, die von Glykolsäure abstammen.

**9.** Material gemäß einem der Ansprüche 1 bis 8, **dadurch gekennzeichnet, dass** das Polymer eine mittlere Molekularmasse hat, die ausreichend hoch ist, damit das Material in der Lage ist, die mechanischen Drucke auszuhalten, dem es nach der Implantierung ausgesetzt werden soll.

**10.** Material gemäß Anspruch 9, **dadurch gekennzeichnet, dass** das Polymer eine mittlere Molekulargewichtsmasse aufweist, die mindestens gleich ungefähr 40.000 ist.

**11.** Material gemäß einem der Ansprüche 1 bis 8, **dadurch gekennzeichnet, dass** das Polymer eine mittlere Molekularmasse hat, die ausreichend gering ist, **dass** das Material bei einer Temperatur unter 50°C, und insbesondere bei 37°C in Form eines teigigen Feststoffs vorliegt.

**12.** Material gemäß Anspruch 11, **dadurch gekennzeichnet, dass** es zumindest teilweise aus einem Polymer mit einer mittleren Molekulargewichtsmasse unter 10.000, und insbesondere unter 5.000 besteht.

**13.** Material gemäß einem der Ansprüche 1 bis 12, **dadurch gekennzeichnet, dass** es 50 bis 70 Gew.% an Calciumcarbonat enthält.

**14.** Material gemäß einem der Ansprüche 1 bis 13, **dadurch gekennzeichnet, dass** das Calciumcarbonat in Form von Calcit oder Aragonit vorliegt.

**15.** Material gemäß einem der Ansprüche 1 bis 14, **dadurch gekennzeichnet, dass** das Calciumcarbonat aus den Skeletten von Meeresorganismen aus Calciumcarbonat oder den Schalen von Mollusken oder Bivalvia erhalten wird.

16. Material gemäß Anspruch 15, **dadurch gekennzeichnet, dass** das Calciumcarbonat aus Korallenskeletten, den Skeletten oder Stacheln von Seeigeln oder den Schalen von Pinctada margaritifera erhalten wird.

17. Material gemäß einem der Ansprüche 1 bis 16, **dadurch gekennzeichnet, dass** es in Form eines formbaren Pulvers oder in Form eines formbaren massiven Teils vorliegt.

18. Verwendung von Calciumcarbonatteilchen, die innerhalb einer Polymermatrix in Anteilen von 40 bis 70% des Gesamtgewichts verteilt sind, zur Herstellung eines Teils einer Knochenprothese, wobei die Teilchengrößen unter 1 mm haben und das Polymer ein Milchsäurepolymer ist.

19. Verwendung gemäß Anspruch 18, **dadurch gekennzeichnet, dass** das Material eines ist, wie in einem der Ansprüche 2 bis 17 definiert.

20. Verfahren zum Verringern der Resorptionsgeschwindigkeit eines Milchsäurepolymers, die eine Matrix in einem Material für eine Knochenprothese bildet, **dadurch gekennzeichnet, dass** man innerhalb der Matrix Calciumcarbonatpartikel verteilt, wobei die Partikelgrößen unter 1 mm haben und 40 bis 70% des Gesamtgewichts der Matrix bilden.

21. Verfahren gemäß Anspruch 20, **dadurch gekennzeichnet, dass** die Polymermatrix so ist, wie in einem der Ansprüche 2 bis 12 definiert.